# EUROPÄISCHE PATENTSCHRIFT

(11) **EP 2 131 801 B1**
(45) Veröffentlichungstag und Bekanntmachung des Hinweises auf die Patenterteilung: **13.04.2011**
(21) Anmeldenummer: 08839357.4
(22) Anmeldetag: 19.10.2008
(51) Int. Cl.: A61F 5/56

(54) **SYSTEM FÜR DIE UNTERKIEFER-PROTRUSION ZUR VERHINDERUNG VON SCHNARCHEN UND APNOE**
SYSTEM FOR MANDIBULAR PROTRUSION TO PREVENT SNORING AND APNEA
SYSTÈME DE PROTRUSION DU MAXILLAIRE INFÉRIEUR POUR EMPÊCHER LE RONFLEMENT ET L'APNÉE

(30) Priorität: 19.10.2007 DE 102007050309; 14.10.2008 DE 102008051221
(43) Veröffentlichungstag der Anmeldung: 16.12.2009
(73) Patentinhaber: Schmitt-Bylandt, Jürgen, 63571 Gelnhausen (DE)
(72) Erfinder: Schmitt-Bylandt, Jürgen, 63571 Gelnhausen (DE)
(74) Vertreter: Wolf, Michael
(86) Internationale Anmeldenummer: PCT/DE2008/001683
(87) Internationale Veröffentlichungsnummer: WO 2009/049602

(56) Entgegenhaltungen:
- WO-A-96/16618
- WO-A-2005/013867
- DE-U1-202007 007 760
- US-A- 5 066 226
- US-A- 6 012 919
- US-A1- 2006 174 897

## Beschreibung

Probleme in Zusammenleben und Partnerschaft durch Schnarchen sind weithin bekannt und die konditionellen Einschränkungen und gesundheitlichen Risiken von dadurch verursachtem, zeitweisen Atemstillstand (Apnoe) sind medizinisch gut dokumentiert [1 bis 4]. Daher hat es zahlreiche Versuche gegeben, Schnarchen technisch oder durch Verhaltensbeeinflussung zu verhindern.

Als generell wirksam haben sich bislang jedoch nur der operative Eingriff zur Verkürzung des Gaumensegels und die Anwendung sogenannter Protrusionsschienen erwiesen, die ein Zurückfallen des Unterkiefers im Schlaf verhindern.

Während ein operativer Eingriff nur in schwerwiegenden Fällen gerechtfertigt erscheint, bringen die bisher angewandten Protrusionsschienen gravierende Beeinträchtigungen bei der Anwendung mit sich, die eine Anwendung auch nach bestmöglicher individueller Anpassung auf Dauer verhindern.

Dabei arbeitet die Mehrzahl der bisher bekannten Verfahren mit je einer auf die Zahnreihe des Ober- und Unterkiefers aufgesetzten Schiene, die über einstellbare Gelenke miteinander so verbunden sind, dass der Unterkiefer bei Entspannung der Kaumuskeln im Schlaf nicht zurückfallen kann.

Dies ist zwar hinsichtlich der Vermeidung des Schnarchens durchaus effektiv. Schwierig ist jedoch, den Gelenkmechanismus so anzupassen, dass natürliche Bewegungen des Unterkiefers nicht behindert werden, insbesondere keine Verspannung der Kiefergelenke und Krämpfe der Kaumuskulatur auftreten. Doch auch bei optimaler Einstellung wird die gewollte Einschränkung der Bewegungsfähigkeit der Kiefer zwangsläufig als beeinträchtigend empfunden. So sind z.B. Gähnen nur eingeschränkt möglich, Sprechen stark erschwert und die Zwangshaltung der Kiefer störend.

Weiter stark beeinträchtigend sind die auf die Zahnreihen der Kiefer aufgesetzten Schienen selbst, die konstruktiv Zahnprothesen ähneln, aber dadurch, dass sie die Kauflächen überdecken, i.d.R. das gewohnte Schließen des Gebisses verhindern. Zudem bringt die Notwendigkeit einer weitgehend festen Verbindung mit den Zahnreihen beider Kiefer mit sich, dass die Schienen in Kontakt mit frei stehenden Zahnhälsen und dem Zahnfleisch kommen und dort Irritationen und gelegentliche Entzündungen hervorrufen.

Es sind dagegen zahlreiche Versuche bekannt, diese Nachteile zu überwinden, die jedoch bislang nur partiell wirksam wurden und daher die Anwendung der Protrusionsschienen faktisch auf extreme Fälle beschränken, bei denen die Beeinträchtigungen als unvermeidbar hingenommen werden müssen:
So wird mit Verfahren, die eine weitgehend feste Verbindung zwischen Ober- und Unterkiefer vorsehen (FR 27 27 008, JP 2004 073 473) die geringste Compliance der Patienten erzielt, Systeme, wie Teil-Bewegungen ermöglichende Klammern nach JP 2006 095 245, die die Zungenbewegung beschränken und ähnlich wie Zahnprothesen in Zahnkronen verklammert werden sollen, finden ebensowenig Akzeptanz.

Mit den Systemen von Schlieper (DE 100 29 875 , DE 102 16 242 und DE 103 31 531) wird versucht, zumindestens den Zungenraum frei zu halten, um einigermaßen ungestörtes Sprechen zu erlauben. Während ersteres noch auf eine weichelastische Verbindung und damit sehr eingeschränkte Bewegung der beidem Schienen zueinander beschreibt, sind die Folgeentwicklungen mit Teleskopartigen, einstellbaren (aber dann starren) Verbindungen (sog. Herner-Teleskop) ausgestattet, die auch die früher verbreiteten Protrusionsschienen nach Hinz (u.a. im Vertrieb der Scheu-Dental, Esslingen) aufwiesen und die oben beschriebenen Nachteile beinhalten.

Ebenfalls den Zungenraum frei halten soll US 4.901.737, die jedoch Unterkiefer-seitig einen Prothese-artigen Einsatz vorsieht, der mit einer offensichtlich kompliziert einzusetzende (und teuer individuell herzustellenden) Metallspange für den Oberkiefer ausgestattet ist.

Weiter haben sich Vorschläge, die ein Zurückfallen der Zunge durch Verklemmen (JP 2005 312 853 und US 2006.130.850, oder durch Vorhalten mit Unterdruck (FR 2 76 94 96) offensichtlich mangels Akzeptanz der Patienten nicht bewährt.

Ebenso problematisch erwiesen sich Systeme, die die Kiefer durch Abstützung im Gaumenraum (US 5.117.816) anpassen oder durch Nockeneinsätze zwischen den Zahnreihen beschränken sollen (US 5.003.994, DE 10 2004 007 008), oder zwar beim Einsetzen verstellbar, aber dann starr sind (wie US 5.570.704), bzw. nur in Längsrichtung beweglich wie in DE 10 2004 058 081, weil dies jeweils einer natürlichen Kieferbewegung widerspricht.

Bessere Akzeptanz finden dagegen Systeme, bei denen der Vorschub des Unterkiefers gegenüber dem Oberkiefer elastisch, etwa durch den Einsatz von Kautschukbändern erfolgt, wie in CA 1998 223 65 03 und US 5.947.724/ 5.794.627, oder in US 5.570.704 beschrieben. Störend sind hier jedoch relativ massive Schienen auf den Zahnreihen und vorstehende Halterungen, in die der Patient die Gummibänder einhaken muss.

Ferner sind in nahezu allen Fällen Spangen vorgesehen, die die Zahnreihen dreiseitig einschlieβen. Doch bis auf die oben genannten Lösungen behindern sie die Zungenbewegung und stören daher massiv beim Sprechen. In jedem Falle werden sie m Mundraum aber als Fremdkörper empfunden und dies um so mehr, je dicker sie sind und je mehr sie Aufdruck auf empfindliche Bereiche, z.B. das Zahnfleisch, ausüben.

Andererseits sind Ausführungen, die zwar das Zahnfleisch durch entsprechend weiche Polsterung schonen, wie US 5.003.994, US 5.829.441 und EP 120 35 70, dafür aber entsprechend dick auftragen, kaum langfristig zu ertragen, weil der Patient den Mund damit nicht schließen kann. Medizinisch strittig, bzw. nur bei Fällen schwerer Apnoe wirklich erforderlich, ist zudem eine zwangsweise Öffnung zwischen den Kiefern zum Lufteintritt, die Patienten als störend empfinden.

Ein System gemäß dem Oberbegriff von Anspruch 1 ist aus der DE-U-20 2007 007 760 bekannt.

Es ist daher Aufgabe vorliegender Erfindung, eine Lösung zu finden, die ein Zurückfallen des Unterkiefers im Schlaf verhindert, den Patienten dabei jedoch nur minimal belastet: Er soll ungehindert sprechen und Gähnen, sowie den Kiefer in alle gewohnte Richtungen bewegen können, die Schienen sollen im Mund kaum stören und auch möglichst unsichtbar sein und die Protrusion des Unterkiefers soll elastisch erfolgen und nur so stark sein, dass eine quasinatürliche Kieferstellung erreicht und empfunden wird.

Dies wird erfindungsgemäß durch das System gemäß Anspruch 1 gelöst. Transparente Schienen, z.B. aus PET können Verwendung finden, die - vorzugsweise durch Tiefziehen- so geformt sind, dass die Auflage auf den Zahnflächen dünn und an den Flanken von nur erforderlicher Stärke sind, der vordere Zungenraum und die Zahnhälse sowie das Zahnfleisch ausgespart bleiben und nur eine federnde Verbindung von Unter- und Oberkiefer die Protrusion bewirkt.

Dazu sind die transparenten Schienen mit zwei Klammern aus federndem Material, vorzugsweise einer Titan-Nickel Memory-Metall-Verbindung, verbunden, die in geeigneten Ausführungen vor-produziert und gegebenenfalls mit Schraubeinsätzen feinjustiert, oder nach individueller Anpassung durch Erhitzen fixiert werden können.

Je nach Kiefer- und Gelenkstellung können diese Klammern als vergleichsweise kurze Bögen ausgeführt sein oder ggf. eine federnde Schleife aufweisen.

Im Falle ungünstiger Zahnstellungen oder Zahnformen, die einen Festen Sitz der Protrusionsschienen behindern (z.B. bei sog. Pyramidenzähnen) ist es ferner möglich, die Schienen mit Mechanismen wie z.B. Haken und Klemmen zu ergänzen, die von Zahn- oder Kiefernspangen bekannt sind, um den Halt in den Zahnreihen zu verbessern.

In einer erweiterten Ausführung dieses Systems (Prio-Anmeldung vom 14.10.2008) wird vorgeschlagen, der natürlichen Kinematik des Unterkiefers weiter zu folgen, indem der Veränderung des Benettwinkels, der sich aus der Lateral- und der Rotationsbewegung der Unterkieferngelenke ergibt, weiter dadurch entsprochen wird, dass die Protusion durch die Vorspannung zwischen Ober- und Unterkiefer von der Rotation des Kiefergelenks durch Einfügen eines weiteren Gelenks entkoppelt wird, das - nahezu parallel zum Kiefergelenk angeordnet - der Verbindung einen zusätzlichen Freiheitsgrad in den Rotationsebenen verleiht.

Durch diese Anordnung, bei der das Schienensystem selbst zum Gelenk wird, ist gewährleistet, dass trotz definierter Protrusion des Unterkiefers die physiologische Belastung von Bandapparatur und Kaumuskulatur gering bleibt.

Zudem werden auch bei weiter Öffnung des Mundes, z.B. beim Gähnen, keine abziehenden Kräfte auf die Schienen ausgeübt. Sie können daher dünner ausgeführt werden und müssen nicht unter Spannung auf die Zahnreihen gepresst werden, was sonst die Wieder-Entnahme erheblich erschwert.

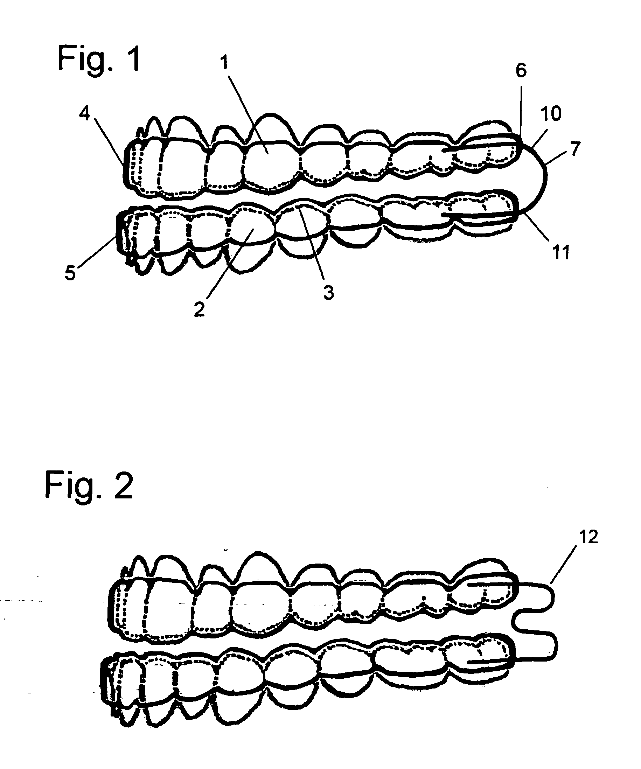

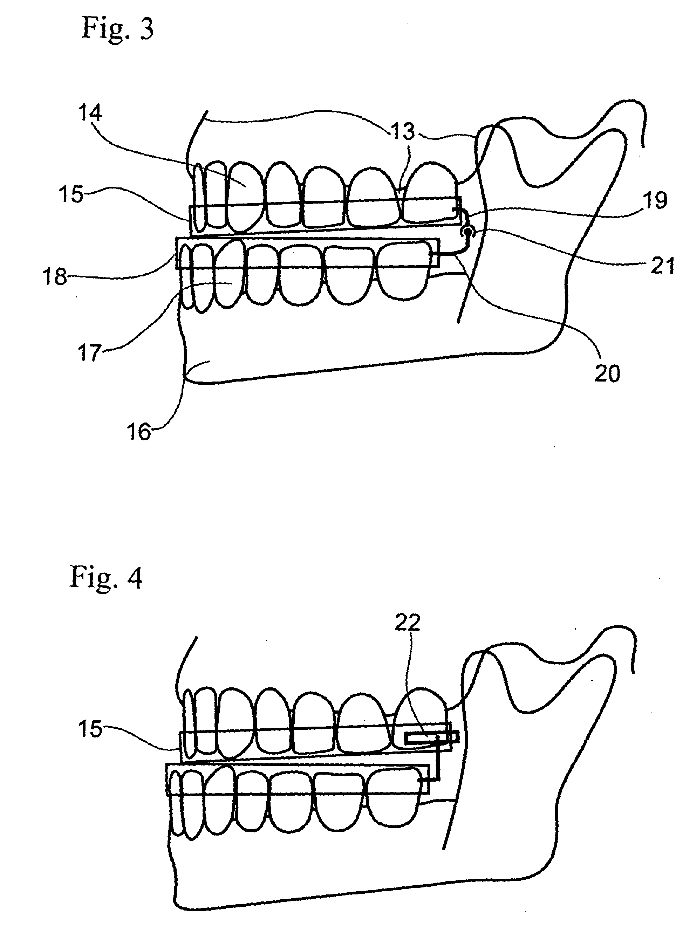

Die Details der Erfindung werden nachfolgend an Hand der Zeichnungen Fig. 1 und 2 näher beschrieben. Dabei zeigen die Zeichnungen Fig. 1 und 2 Ausführungen mit federnden Klammern oder Bügeln, während Fig. 3 bis 7 die zusätzliche Einführung von Gelenken beschreibt.

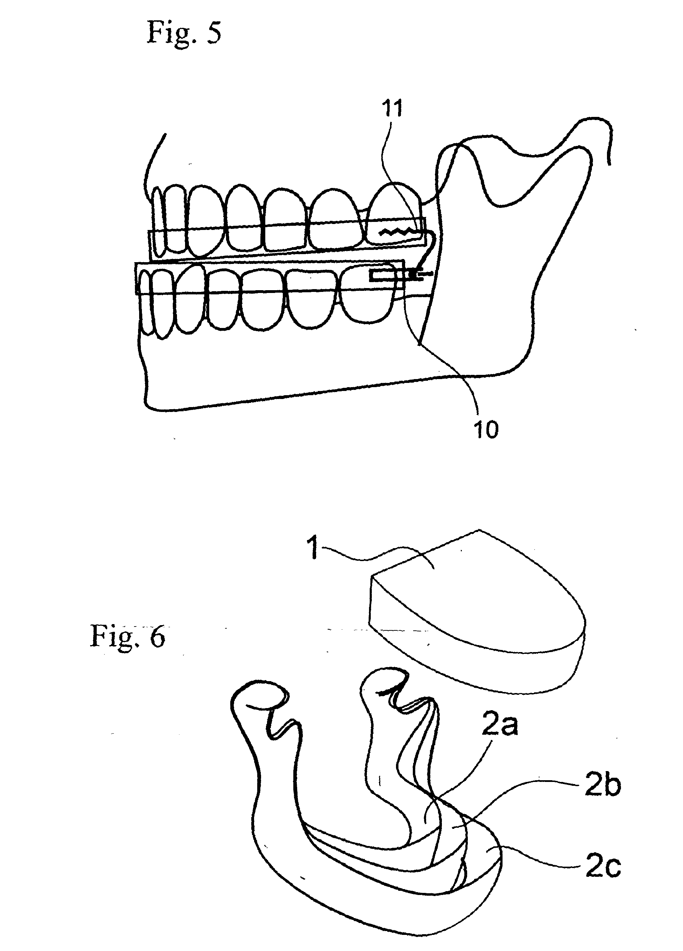

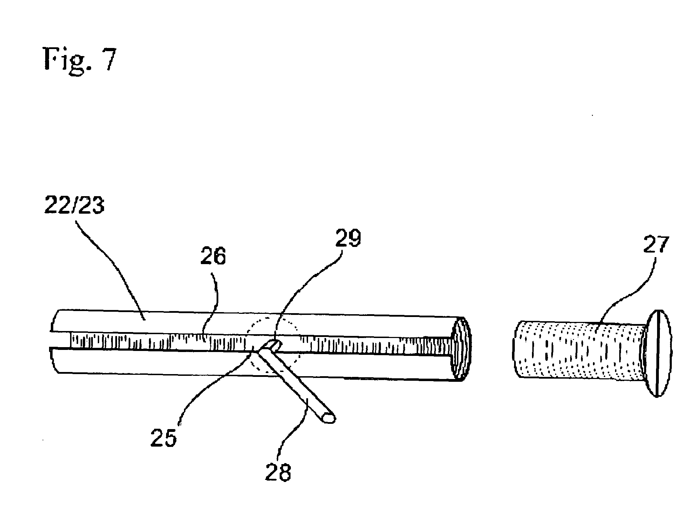

Dabei stellt Fig. 4 bis 6 verschiedene Anlagen des System in Seitenansicht dar und Fig. 6 ver schiedene Bewegungsphasen des Kiefers dreidimensional, während Fig. 7 die Verstellmöglichkeit des Gelenks in Fig. 5 und 6 im Detail darstellt.
**Fig.** 1 Zeigt die Oberkieferspange **1** und Unterkieferspange **2,** wobei das Material über den Kauflächen **3** durch selektiv stärkere Erhitzung des Tiefziehmaterials dünner ist als an den Flanken **4** bis **6**. Die Metallbügel **7** (hier exemplarisch kurz ausgebildet), können in verschiedenen, für unterschiedliche Gebißstellungen ausgeführten Versionen vorbereitet sein und in die Gebißschienen eingeschmolzen oder einpolymerisiert werden.
   Weiter ist es möglich, die Bereiche **10** und **11** der Bügel nach der Anbringung von Markierungen zur Kennzeichnung der Normalstellung des Gebisses zu erhitzen und so auszurichten und abzugekühlen, dass der gewünschte Vorschub erreicht wird.

**Fig. 2** zeigt die gleiche Anordnung, jedoch mit den einen weiteren Federbereich ermöglichenden, spiralig gewundenen Metallbügeln **12,** die insbesondere dann einzusetzen sind, wenn ein weiter Bewegungsbereich erforderlich ist.
**Fig. 3** zeigt den Oberkiefer **13** mit der oberen Zahnreihe **14** und der darauf eingerasteten Schien ne **15** und dazu den Unterkiefer **16** in vorgeschobenen Stellung, wiederum mit Zahnreihe **17** und Schiene **18,** die durch eine federnde Verbindung über die Klammern **19** und **20** verbunden sind Dabei sind die Klammern **19** und **20** durch das Gelenk **21** verkoppelt.
**Fig. 4** zeigt die gleiche Anordnung mit einem am Ende der oberen Schiene **15** angebrachten, einstellbaren Gelenk **22,** das in Fig. **7** detailliert dargestellt ist.
**Fig. 5** stellt die gleiche Anordnung dar, nur ist hier das verstellbare Gelenk **23** an der unteren Schiene **18** befestigt.
**Fig. 6** zeigt die Bewegungsphasen dieser Anordnungen in den Stellungen des Oberkiefers **1 a, b und c,** sowie analog des Unterkiefers **2 a, b und c-** hier allerdings ohne die Federwirkungen der Klammern **18** und **19** zu berücksichtigen. Wie zu erkennen ist, kann der Unterkiefer trotz Protrusion weitgehend unbeeinträchtigt Kaubewegungen ausführen.
**Fig 7** zeigt das Detail eines verstellbaren Gelenks **22 oder 23,** wie in **Fig. 4** **und** **5** beschrieben. Dabei wird eine Kugel **25** in einer geschlitzten Hülse 26 geführt und über die Stellschraube **27** justiert. In die Kugel **25** ist ein Arm **28** eingesetzt, der im Schlitz **29** der Hülse **26** mit der Kugel verschiebbar ist und die Verbindung zur Schiene der jeweils anderen Zahnreihe darstellt.

### Literatumachweis:

[1] American Academy of Dental Sleep Medicine ADSM, Kongressbericht, Philadelphia 2004, in Somnojoumal 3/04
[2] An American Sleep Disorder Associations Report in SLEEP 1995, 18 (6), S. 511 ff.
[3] Hein, Rascke, Köhler, Mayer, Peter und Rühle: Leitlinie zur Diagnostik und Therapie schlafbezogener Atmungsstörungen beim Erwachsenen. Pneumaologie 2001/55, S. 339 ff.
[4] Cartwright & Samuelson: The Effects of a nonsurgical Treatment for Obstructive Sleep Apnea. JAMA, Aug. 1982, Vol. 248, Nr. 6, S. 705 ff.

## Patentansprüche

1. System für die Unterkiefer-Protrusion zur Verhinderung von Schnarchen und Apnoe, unit Schienen (1, 2, 15, 18) zur Fanung von Oberkiefer und Unterkiefer wobei die Schienen (1, 2, 15, 18) mit Klammern aus federndem Material (7, 19, 20) verbunden sind, die den Vorschub des Unterkiefers bewirkten,
**dadurch gekennzeichnet,**
**dass** die Klammern (7, 19, 20) an den hinteren Enden der Schienen (1, 2, 15, 18) angeordnet sind.

2. System für die Unterkiefer-Protrusion zur Verhinderung von Schnarchen und Apnoe nach Anspruch 1,
**dadurch gekennzeichnet,**
**dass** dass die Schienen aus PET oder ähnlichem Kunststoffmaterial bestehen.

3. System für die Unterkiefer-Protrusion zur Verhinderung von Schnarchen und Apnoe nach Anspruch 1 oder 2,
**dadurch gekennzeichnet,**
**dass** die Schienen mit Klammern aus Nickel-Titan-Legierungen verbunden sind, wobei die Klammern vorzugsweise Memory-Metall-Effekte aufweisen und mittels Hitze justiert werden.

4. System für die Unterkiefer-Protrusion zur Verhinderung von Schnarchen und Apnoe nach Anspruch 1 bis 3,
**dadurch gekennzeichnet,**
**dass** die Klammern in verschiedenen Formen und Ausführungen vorgehalten und jeweils nach Bedarf mit den Schienen kombiniert werden, wobei die gewählten Klammern vorzugsweise in die Schienen einpolymerisiert werden.

5. System für die Unterkiefer-Protrusion zur Verhinderung von Schnarchen und Apnoe nach Anspruch 4,
**dadurch gekennzeichnet,**
**dass** die Schienen zylinderförmige Aufnahmen aufweisen, in denen die Klammern in unterschiedlicher Tiefe fixiert werden, wobei die Klammern vorzugs- und wahlweise mit seitlichen Fixierschrauben befestigt oder mit Überwurfmuttern fixiert sind.

6. System für die Unterkiefer-Protrusion zur Verhinderung von Schnarchen und Apnoe nach Anspruch 4 und 5,
**dadurch gekennzeichnet,**
**dass** die Klammern oder ihre Haltevorrichtungen aus den Schienen etwas vorstehen, so dass die Schienen an diesen Überständen leicht abgezogen und aus dem Mund entfernt werden können.

7. System für die Unterkiefer-Protrusion zur Verhinderung von Schnarchen und Apnoe nach Anspruch 1,
**dadurch gekennzeichnet,**
**dass** die Schienen im Bereich des vorderen Gaumens so weit ausgeschnitten sind, dass sie weitgehend ungestört Zungenbewegungen und damit unbehindertes Sprechen ermöglichen.

8. System für die Unterkiefer-Protrusion zur Verhinderung von Schnarchen und Apnoe nach Anspruch 1,
**dadurch gekennzeichnet,**
**dass** die Klammern aus dünnem, aber steifen Material gefertigt sind, dass die Zahnhälse nicht ganz bedeckt.

9. System für die Unterkiefer-Protrusion zur Verhinderung von Schnarchen und Apnoe nach Anspruch 1,
**dadurch gekennzeichnet,**
**dass** die Klammern auf den Kauflächen besonders dünn ausgeführt sind, um das Schließen der Kiefer gegeneinander nicht zu behindern.

10. System für die Unterkiefer-Protrusion zur Verhinderung von Schnarchen und Apnoe nach Anspruch 1,
**dadurch gekennzeichnet,**
**dass** die Schienen im Bedarfsfall zusätzliche Haltevorrichtungen an und zwischen den Zähnen aufweisen, wenn besondere Zahnformen (z. B. Pyramidenzähne) oder Zahnstellungen dies erfordern, wobei diese Haltevorrichtungen vorzugsweise einzelne Zähne umgreifende Klammern sind.

11. System für die Unterkiefer-Protrusion zur Verhinderung von Schnarchen und Apnoe nach Anspruch 1,
**dadurch gekennzeichnet,**
**dass** die Bügel oder Klammern, die die Schienen federnd verbinden, ihrerseits jeweils mit einem Gelenk verbunden sind,
das eine Bewegung der Kiefer in lateraler und rotativer Richtung ohne Gegendruck durch die Bügel oder Klammern ermöglicht.

12. System für die Unterkiefer-Protrusion zur Verhinderung von Schnarchen und Apnoe nach Anspruch 11,
**dadurch gekennzeichnet,**
**dass** die Gelenke im Mundinneren parallel zur Achse der Kieferngelenke angeordnet sind.

13. System für die Unterkiefer-Protrusion zur Verhinderung von Schnarchen und Apnoe nach Anspruch 11,
**dadurch gekennzeichnet,**
**dass** die Gelenke wahlweise jeweils mittig zwischen je zwei Bügelelementen, an den hinteren Enden der oberen Schienen oder an den hinteren Enden der unteren Schienen angeordnet sind.

14. System für die Unterkiefer-Protrusion zur Verhinderung von Schnarchen und Apnoe nach Anspruch 11,
**dadurch gekennzeichnet,**
**dass** der Grad der Protrusion durch Verschiebung der Bügel in einer Gleithülse durch eine Stellschraube justierbar ist.

15. System für die Unterkiefer-Protrusion zur Verhinderung von Schnarchen und Apnoe nach Anspruch 11,
**dadurch gekennzeichnet,**
**dass** eine Gelenkkugel in einer leicht nach innen geneigten Hülse so geführt ist, dass sich zusätzlicher Spielraum für eine Bewegung nach vorne und seitwärts ergibt.

## Claims

1. System for mandibular protrusion to prevent snoring and apnoea, with splints (1, 2, 15, 18) for holding the maxilla and mandible, wherein the splints (1, 2, 15, 18) are connected with clamps of elastic material (7, 19, 20) which bring about the forward thrust of the mandible,
**characterized in that**
the clamps (7, 19, 20) are arranged at the rear ends of the splints (1, 2, 15, 18).

2. System for mandibular protrusion to prevent snoring and apnoea according to Claim 1,
**characterized in that**
the splints consist of PET or similar plastic material.

3. System for mandibular protrusion to prevent snoring and apnoea according to Claim 1 or 2,
**characterized in that**
the splints are connected with clamps of nickel-titanium alloys, wherein the clamps preferably have memory metal effects and are adjusted by means of heat.

4. System for mandibular protrusion to prevent snoring and apnoea according to Claim 1 to 3,
**characterized in that**
the clamps are kept in various shapes and embodiments and are combined with the splints respectively as required, wherein the selected clamps are preferably polymerised into the splints.

5. System for mandibular protrusion to prevent snoring and apnoea according to Claim 4,
**characterized in that**
the splints have cylindrical mountings in which the clamps are fixed at differing depths, wherein the clamps are preferably and optionally fastened with lateral fixing screws or with union nuts.

6. System for mandibular protrusion to prevent snoring and apnoea according to Claim 4 and 5,
**characterized in that**
the clamps or their holding devices project a little from the splints, so that the splints can be easily withdrawn at these overhangs and removed from the mouth.

7. System for mandibular protrusion to prevent snoring and apnoea according to Claim 1,
**characterized in that**
the splints are cut out in the region of the front palate to such an extent that they largely make possible unimpeded movements of the tongue and hence unimpeded speech.

8. System for mandibular protrusion to prevent snoring and apnoea according to Claim 1,
**characterized in that**
the clamps are made from thin but rigid material which does not entirely cover the tooth necks.

9. System for mandibular protrusion to prevent snoring and apnoea according to Claim 1,
**characterized in that**
the clamps are constructed so as to be particularly thin on the masticatory surfaces, so as not to impede the closing of the jawbones with respect to each other.

10. System for mandibular protrusion to prevent snoring and apnoea according to Claim 1,
**characterized in that**
if necessary the splints have additional holding devices on and between the teeth, if particular tooth shapes (e.g. pyramid teeth) or tooth positions require this, wherein these holding devices are preferably clamps embracing individual teeth.

11. System for mandibular protrusion to prevent snoring and apnoea according to Claim 1,
**characterized in that**
the brackets or clamps which connect the splints elastically are in turn respectively connected with a joint which makes possible a movement of the jawbones in lateral and rotative direction without counterpressure by the brackets or clamps.

12. System for mandibular protrusion to prevent snoring and apnoea according to Claim 11,
**characterized in that**
the joints are arranged in the interior of the mouth paralel to the axis of the temperomandibular joints.

13. System for mandibular protrusion to prevent snoring and apnoea according to Claim 11,
**characterized in that**
the joints are arranged optionally respectively centrally between two bracket elements in each case, at the rear ends of the upper splints or at the rear ends of the lower splints.

14. System for mandibular protrusion to prevent snoring and apnoea according to Claim 11,
**characterized in that**
the degree of protrusion is adjustable by displacement of the brackets in a sliding sleeve by a setscrew.

15. System for mandibular protrusion to prevent snoring and apnoea according to Claim 11,
**characterized in that**
a joint ball is guided in a slightly inwardly inclined sleeve so that additional play is produced for a movement forwards and sideways.

## Revendications

1. Système pour la protusion de la mâchoire inférieure destiné à empêcher le ronflement et l'apnée avec des rails (1, 2, 15, 18) pour saisir les mâchoires supérieure et inférieure, sachant que les rails (1, 2, 15, 18) sont reliés par des fixations en matériau élastique (7, 19, 20) qui effectuent l'avancement de la mâchoire inférieure,
**caractérisé en ce que**
les fixations (7, 19, 20) sont placées sur les extrémités arrière des rails (1, 2, 15, 18).

2. Système pour la protusion de la mâchoire inférieure destiné à empêcher le ronflement et l'apnée selon la revendication 1,
**caractérisé en ce que**
les rails sont en PET ou matériau plastique similaire.

3. Système pour la protusion de la mâchoire inférieure destiné à empêcher le ronflement et l'apnée selon la revendication 1 ou 2,
**caractérisé en ce que**
les rails sont reliés par des fixations en alliages de nickel-titane,sachant que les fixations présentent de préférence des effets mémoire métalliques et sont ajustées par la chaleur.

4. Système pour la protusion de la mâchoire inférieure destiné à empêcher le ronflement et l'apnée selon l'une des revendications 1 à 3,
**caractérisé en ce que**
les fixations sont présentées en différentes formes et réalisations et peuvent être combinées avec les rails selon les besoins, sachant que les fixations choisies sont de préférence polymérisées dans les rails.

5. Système pour la protusion de la mâchoire inférieure destiné à empêcher le ronflement et l'apnée selon la revendication 4,
**caractérisé en ce que**
les rails présentent des réceptions cylindriques dans lesquelles les rails sont fixés avec différentes profondeurs, sachant que les fixations sont fixées avec de préférence et au choix des vis de fixation latérales ou des collerettes de fixation.

6. Système pour la protusion de la mâchoire inférieure destiné à empêcher le ronflement et l'apnée selon les revendications 4 et 5,
**caractérisé en ce que**
les fixations ou leurs dispositifs de maintien dépassent quelque peu des rails, de façon à ce que les rails soient légèrement en retrait sur ces saillies et puissent être éloignés de la bouche.

7. Système pour la protusion de la mâchoire inférieure destiné à empêcher le ronflement et l'apnée selon la revendication 1,
**caractérisé en ce que**
les rails sont ainsi découpés si loin au niveau du palais dur qu'ils permettent sans gêne et dans une large mesure les mouvements de la langue et la parole.

8. Système pour la protusion de la mâchoire inférieure destiné à empêcher le ronflement et l'apnée selon la revendication 1,
**caractérisé en ce que**
les fixations sont fabriquées dans un matériau fin mais rigide qui ne recouvre pas totalement le collet des dents.

9. Système pour la protusion de la mâchoire inférieure destiné à empêcher le ronflement et l'apnée selon la revendication 1,
**caractérisé en ce que**
les fixations sont particulièrement fines sur les surfaces de mastication afin de ne pas empêcher la fermeture des mâchoires l'une contre l'autre.

10. Système pour la protusion de la mâchoire inférieure destiné à empêcher le ronflement et l'apnée selon la revendication 1,
**caractérisé en ce que**
les rails présentent si besoin des dispositifs de maintien supplémentaires sur et entre les dents lorsque des formes de dents particulières (dents en pyramide par ex.) ou des positions de dents l'exigent, sachant que ces dispositifs de maintien sont de préférence des fixations entourant des dents individuelles.

11. Système pour la protusion de la mâchoire inférieure destiné à empêcher le ronflement et l'apnée selon la revendication 1,
**caractérisé en ce que**
les étriers ou les fixations qui relient les rails de manière élastique, sont reliés pour leur part à une articulation qui permet un mouvement latéral et une rotation de la mâchoire sans contre-pression par les étriers ou les fixations.

12. Système pour la protusion de la mâchoire inférieure destiné à empêcher le ronflement et l'apnée selon la revendication 11,
**caractérisé en ce que**
les articulations sont placées à l'intérieur de la bouche parallèlement à l'axe des articulations de la mâchoire.

13. Système pour la protusion de la mâchoire inférieure destiné à empêcher le ronflement et l'apnée selon la revendication 11,
**caractérisé en ce que**
les articulations sont placées au choix au centre respectivement entre deux éléments d'étrier, sur les extrémités arrière des rails supérieurs ou sur les extrémités arrière des rails inférieurs.

14. Système pour la protusion de la mâchoire inférieure destiné à empêcher le ronflement et l'apnée selon la revendication 11,
**caractérisé en ce que**
le degré de protusion peut être réglé par un avancement des étriers dans un manchon de coulissement par une vis de réglage.

15. Système pour la protusion de la mâchoire inférieure destiné à empêcher le ronflement et l'apnée selon la revendication 11,
**caractérisé en ce qu'**
une bille d'articulation est ainsi dirigée dans un manchon légèrement incliné vers l'intérieur que cela crée une marge supplémentaire pour un mouvement avant et latéral.
